# EUROPÄISCHE PATENTSCHRIFT

(11) **EP 1 418 169 B1**
(45) Veröffentlichungstag und Bekanntmachung des Hinweises auf die Patenterteilung: **25.02.2009**
(21) Anmeldenummer: 03024919.7
(22) Anmeldetag: 29.10.2003
(51) Int. Cl.: C07C 209/68, C07C 211/52, C07C 231/12, C07C 233/15

(54) **Verfahren zur Herstellung von Polyhalogenalkylaryl-Verbindungen**
Process for the preparation of polyhalogenoalkylaryl compounds
Procédé de préparation de dérivés d'alkylaryle polyhalogenés

(30) Priorität: 11.11.2002 DE 10252273
(43) Veröffentlichungstag der Anmeldung: 12.05.2004
(73) Patentinhaber: Saltigo GmbH, 40764 Langenfeld (DE)
(72) Erfinder: Marhold, Albrecht, Dr., 51373 Leverkusen (DE); Pleschke, Axel, Dr., 51069 Köln (DE)
(74) Vertreter: Wichmann, Birgid

(56) Entgegenhaltungen:
- EP-A- 0 298 803
- EP-A- 1 006 102
- DE-A- 1 911 610
- DATABASE CROSSFIRE BEILSTEIN [Online] Beilstein Institut zur Förderung der Chemischen Wissenschaften, Frankfurt am Main, DE; XP002272731 Database accession no. BRN 6707222, 2660677 & KNUNYANTS, I. L. ET AL.: BULL. ACAD. SCI. USSR DIV. CHEM. SCI. (ENGL. TRANSL.), 1962, Seiten 633-640,
- DATABASE CROSSFIRE BEILSTEIN [Online] Beilstein Institut zur Förderung der Chemischen Wissenschaften, Frankfurt am Main, DE; XP002272733 Database accession no. BRN 2983575 & KLABUNDE, K. J.; BURTON, D. J.: J. AMER. CHEM. SOC., Bd. 94, 1972, Seiten 820-28,
- DATABASE CROSSFIRE BEILSTEIN [Online] Beilstein Institut zur Förderung der Chemischen Wissenschaften, Frankfurt am Main, DE; XP002272732 Database accession no. Reaction ID 3925708 & UMEMOTO, TERUO; MIYANO OSAMU: BULL. CHEM. SOC., Bd. 57, Nr. 11, 1984, Seiten 3361-62,
- DATABASE CROSSFIRE BEILSTEIN [Online] Beilstein Institut zur Förderung der Chemischen Wissenschaften, Frankfurt am Main, DE; XP002272734 Database accession no. BRN 1873945 & JAKUBOWITSCH ET AL.: ZH. VSES. KHIM. O'VA IM. D.I. MENDELEEVA; 6, 1962, Seiten 709-711,

## Beschreibung

Die vorliegende Erfindung betrifft ein Verfahren zur Herstellung von Polyhalogenalkylarylen, welche zur Herstellung von Wirkstoffen insbesondere in Agrochemikalien und Arzneimitteln Anwendung finden können.

Polyhalogenalkylaryle sind wertvolle Ausgangsprodukte für die Herstellung von Wirkstoffen in Agrochemikalien und Arzneimitteln, da die Polyhalogenalkylsubstituenten die Lipophilie und damit die Membrangängigkeit des gesamten Wirkstoffmoleküls erhöhen. So eignen sich beispielsweise substituierte 4-Perfluoralkylaniline besonders zur Herstellung von wirksamen Insektiziden des Aroylharnstoff-Typs (siehe auch EP-A 919 542 und EP-A 936 212).

Die Herstellung von Perfluoralkylarylen kann beispielsweise durch Umsetzung von Aromaten mit Perfluoralkyliodiden oder -bromiden in aprotischen Lösungsmitteln und entweder in Gegenwart von Metallen und Schwefeldioxid (EP-A 206 951 und FR-A 2 660 923) oder in Gegenwart von Alkalimetalldithionit erfolgen (EP-A 298 803). Analog können Perfluoralkylchloride in Dimethylsulfoxid umgesetzt werden (Huang et al., J. Fluorine Chem., 111, 2001, 107-113).

Nachteilig an den genannten Methoden ist, dass die Umsetzung in einem aprotisch polaren Lösungsmittel wie insbesondere Dimethylformamid oder Dimethylsulfoxid erfolgen muss, die aufgrund ihres hohen Siedepunktes schwer von den Produkten abtrennbar und kaum rückführbar sind und darüber hinaus physiologisch bedenklich sind. Weiterhin weisen alle Methoden lediglich mäßige Ausbeuten auf.

In einem Verfahren gemäß EP-A 1 006 102 können Perfluoralkylaniline durch Umsetzung von Anilinen mit Perfluoralkyliodiden in einem Zweiphasensystem in Gegenwart eines Reduktionsmittels erhalten werden. Allerdings sind die Perfluoralkyliodide nicht nur teuer sondern verursachen aufgrund des hohen Molgewichts auch eine geringe Atomökonomie.

Es bestand daher auch das Bedürfnis, ein Verfahren bereitzustellen, das die Herstellung von Polyhalogenalkylarylen in guten Ausbeuten und in einfacher Weise ermöglicht.

Es wurde nun ein Verfahren zur Herstellung von Verbindungen der Formel (I) gefunden, 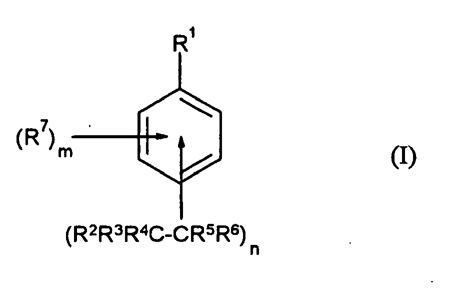 in der
- R¹: für C₁-C₁₂-Alkyl, NR⁸R⁹ oder OR¹⁰ steht, wobei R⁸, R⁹ und R¹⁰ jeweils unabhängig voneinander für Wasserstoff, C₁-C₁₂-Alkyl, CO(C₁-C₁₂-Alkyl), CO(C₅-C₁₄-Aryl), CO(C₆-C₁₅-Arylakyl), COO(C₁-C₁₂-Alkyl), COO(C₅-C₁₄-Aryl), COO(C₆-C₁₅-Arylalkyl), COO(C₂-C₁₂-Alkenyl), CONH(C₁-C₁₂-Alkyl), CONH(C₅-C₁₄-Aryl), CONH(C₆-C₁₅-Arylalkyl), CON(C₁-C₁₂-Alkyl)₂, CON(C₅-C₁₄-Aryl)₂, CON(C₆-C₁₅-Arylalkyl)₂ oder C₆-C₁₅-Arylalkyl stehen, oder NR⁸R⁹ als Ganzes für einen cyclischen Rest mit insgesamt 4 bis 16 Kohlenstoffatomen steht und
- R², R³, R⁴, R⁵ und R⁶: jeweils unabhängig für Wasserstoff, Fluor, Chlor, Brom oder C₁-C₁₂-Polyfluoralkyl stehen und/oder jeweils zwei der Reste R², R³, R⁴, R⁵ und R⁶ einen oder mehrere cyclische Polyfluoralkylreste mit jeweils insgesamt 4 bis 20 Kohlenstoffatomen bilden, wobei für alle Fälle die Auflage gilt, dass die Summe der Fluoratome am Kohlenstoffatom, das die Bindung zum aromatischen Ring herstellt und den Fluoratomen an dem oder den dazu benachbarten Kohlenstoffatomen mindestens zwei beträgt und
- n: für eins oder zwei steht und
- R⁷: für C₁-C₁₂-Alkyl, C_{S}-C₁₄-Aryl, C₆-C₁₅-Arylalkyl, Hydroxy, Chlor, Brom, Fluor, Nitro, Cyano, freies oder geschütztes Formyl, C₁-C₁₂-Halogenalkyl, oder Reste der Formeln (IIa) bis (IIf) steht,

A-B-D-E (IIa)

A-E (IIb)

A-SO₂-E (IIc)

A-B-SO₂R¹¹ (IId)

A-SO₃W (IIe)

A-COW (IIf)
in denen unabhängig voneinander
- A: fehlt oder für einen C₁-C₈-Alkrlenrest steht und
- B: fehlt oder für Sauerstoff, Schwefel oder NR¹² steht,
wobei
R¹² Wasserstoff, C₁-C₈-Alkyl, C₆-C₁₅-Arylalkyl oder C₅-C₁₄-Aryl bedeutet und
- D: für eine Carbonyl-Gruppe steht und
- E: für R¹³, OR¹³, NHR¹¹ oder N(R¹¹)₂ steht,
wobei
R¹³ für C₁-C₈-Alkyl, C₆-C₁₅-Arylalkyl oder C₅-C₁₄-Aryl und
R¹¹ jeweils unabhängig für C₁-C₈-Alkyl, C₆-C₁₅-Arylalkyl oder C₆-C₁₄-Aryl steht oder N(R¹¹)₂ zusammen für einen cyclischen Aminorest mit 4 bis 12 Kohlenstoffatomen steht und
- W: für OH, NH₂, oder OM steht, wobei M ein Alkalimetallion, ein halbes Äquivalent eines Erdalkalimetallions, ein Ammoniumion oder ein organisches Ammoniumion bedeuten kann oder
jeweils zwei Reste R⁷ zusammen einen cyclischen Rest mit insgesamt 5 bis 12 Kohlenstoffatomen bilden können und
- m: für eine ganze Zahl von 0 bis 5-n steht,
das dadurch gekennzeichnet ist, dass
Verbindungen der Formel (II) 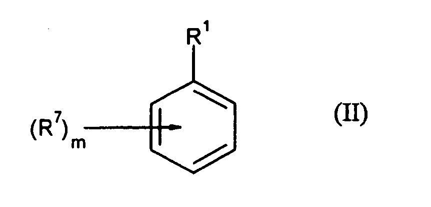 in der
R¹, R⁷ und m die vorstehend genannte Bedeutung besitzen,
mit Verbindungen der Formel (III) umgesetzt werden, 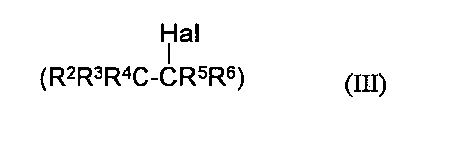 in der
R², R³, R⁴, R⁵ und R⁶ die vorstehend genannte Bedeutung besitzen und
Hal für Brom oder Chlor, bevorzugt für Brom steht und wobei die Umsetzung
- in einem mehrphasigen Reaktionsmedium, das eine wässrige Phase und zumindest eine, bevorzugt genau eine, organische Phase aufweist und
- in Gegenwart von Phasentransferkatalysator und
- in Gegenwart eines Reduktionsmittels und/oder Licht mit einer Wellenlänge von 400 nm oder weniger und
- gegebenenfalls in Gegenwart von Base erfolgt.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Alkyl beziehungsweise Alkylen beziehungsweise Alkoxy beziehungsweise Alkenyl bedeutet jeweils unabhängig einen geradkettigen, cyclischen, verzweigten oder unverzweigten Alkyl- beziehungsweise Alkylen- beziehungsweise Alkoxy- beziehungsweise Alkenyl-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl darüber hinaus beispielsweise für Adamantyl, n-Nonyl, n-Decyl und n-Dodecyl.

C₁-C₈-Alkoxy steht beispielsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

C₂-C₂₀-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 1-Octenyl oder 2-Octenyl.

**Polyfluoralkyl** steht jeweils unabhängig für einen geradkettigen, cyclischen, verzweigten oder unverzweigten Alkylrest, der durch mindestens zwei Fluoratome und gegebenenfalls weiter durch Chloratome und/oder Bromatome substituiert ist.

Beispielsweise steht C₁-C₁₂-Polyfluorakyl für Trifluormethyl, Difluorchlormethyl, Pentafluorethyl, 1,1-Dichlor-2,2,2-trifluorethyl, Heptafluorisopropyl, n-Nonafluorbutyl, Perfluorcyclopentyl, Perfluorcyclohexyl und Perfluordodecyl.

**Perfluoralkyl** steht jeweils unabhängig einen geradkettigen, cyclischen, verzweigten oder unverzweigten Alkylrest, der vollständig durch Fluoratome substituiert ist.

**Aryl** bedeutet jeweils unabhängig einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 14 Gerüstkohlenstoffatomen.

Beispiele für carbocyclische aromatische Reste mit 6 bis 14 Gerüstkohlenstoffatomen sind zum Beispiel Phenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-C₁₂-Alkyl, C₁-C₁₂-Perfluoralkyl, COO(C₁-C₈-Alkyl), CON(C₁-C₈-Alkyl)₂, COO(C₁-C₈-Arylalkyl), COO(C₄-C₁₄-Aryl), CO(C₁-C₈-Alkyl), C₅-C₁₅-Arylalkyl oder Tri(C₁-C₆-alkyl)siloxyl.

Arylalkyl bedeutet jeweils unabhängig einen geradkettigen, cyclischen, verzweigten oder unverzweigten Alkyl-Rest, der einfach, mehrfach oder vollständig durch ArylReste gemäß obiger Definition substituiert sein kann.

C₆-C₁₅-Arylalkyl steht beispielsweise und bevorzugt für Benzyl.

Im Folgenden werden die bevorzugten Substitutionsmuster für Verbindungen der Formeln (I), (II) und (III) definiert:
- R¹: steht bevorzugt für NR⁷R⁸, wobei NR⁷R⁸ weiter bevorzugt als Ganzes für NH₂ oder NHCO(C₁-C₁₂-Alkyl) und noch weiter bevorzugt für NH₂ steht.
R², R³, R⁴, R⁵ und R⁶ stehen bevorzugt für Wasserstoff, Chlor, Fluor oder C₁-C₄-Perfluoralkyl oder R²R³R⁴C-CR⁵R⁶ als Ganzes für einen cyclischen Polyfluoralkylrest mit insgesamt 4 bis 12 Kohlenstoffatomen.

Besonders bevorzugt steht R²R³R⁴C-CR⁵R⁶ als Ganzes für Heptafluor-2-propyl, 1-Brom-1,1,2,3,3,3-hexafluor-2-propyl, 2-Brom-1,1,2,2-tetrafluorethyl, 2-Chlor-1,1,2,2-tetrafluorethyl, 1-Chlor-1,1,2,3,3,3-hexafluor-2-propyl, 2-Brom-2-chlor-trifluorethyl, 2-Brom-1-chlor-trifluorethyl, 3-Brom-2,3-dichlor-1,1,1,4,4,4-hexafluor-2-butyl, 2-Chlor-3,3,4,4-tetrafluor-cyclobutyl, 2-Brom-3,3,4,4-tetrafluor-cyclobutyl 2-Chlor-3,3,4,4,5,5-hexafluor-cyclopentyl und 2-Brom-3,3,4,4,5,5-hexafluor-cyclo-pentyl.
- n: steht bevorzugt für 1.
- R⁷: steht bevorzugt jeweils unabhängig für C₁-C₄-Alkyl, Chlor, Fluor, Nitro, Cyano oder C₁-C₄-Alkoxy, besonders bevorzugt für Methyl, Ethyl, Methoxy oder Ethoxy, ganz besonders bevorzugt für Methyl.
- m: steht bevorzugt für 1 oder 2, besonders bevorzugt für 1.

Besonders bevorzugte Verbindungen der Formel (I) sind 2-Methyl-4-(heptafluor-2-propyl)-anilin, N,2-Dimethyl-4-(1,1,1,2,3,3,3-heptafluor-2-propyl)-anilin, 2-Methyl-4-(1-brom-1,1,2,3,3,3-hexafluor-2-propyl)-anilin, 2-Methyl-4-(2-brom-1,1,2,2-tetrafluorethyl)-anilin, 2-Methyl-4-(2-chlor-1,1,2,2-tetrafluorethyl)-anilin, 2-Methyl-4-(1-chlor-1,1,2,3,3,3-hexafluor-2-propyl)-anilin, 2-Methyl-4-(2-brom-2-chlor-trifluorethyl)-anilin, 2-Methyl-4-(2-brom-1-chlor-trifluorethyl)-anilin, 2-Methyl-4-(3-brom-2,3-dichlor-1,1,1,4,4,4-hexafluor-2-butyl)-anilin, 2-Methyl-4-(2-chlor-3,3,4,4-tetrafluor-cyclobutyl)-anilin, 2-Methyl-4-(2-chlor-3,3,4,4,5,5-hexafluor-cyclopentyl)-anilin, 2-Methyl-4-(2-brom-3,3,4,4-tetrafluor-cyclobutyl)-anilin, 2-Methyl-4-(2-brom-3,3,4,4,5,5-hexafluor-cyclopentyl)-anilin, Essigsäure-2-methyl-4-(1-brom-1,1,2,3,3,3-hexafluor-2-propyl)-anilid, 2-Methyl-4-(2-brom-1,1,1,2,3,4,4,4-octafluor-3-butyl)-anilin und 2-Methyl-4-(2-brom-2,3,3,4,4,5,5-octafluorcyclo-1-pentyl)-anilin.

Bevorzugte Verbindungen der Formel (III) sind Heptafluor-2-brom-propan, Hepta-fluor-2-chlor-propan, 1,2-Dibromtetrafluorethan, 1,2-Dibrom-1-chlor-trifluorethan, 2,3-Dibrom-octafluorbutan, 2,3-Dibrom-2,3-dichlorhexafluorbutan, 2,3-Dibrom-2,3-dichlorhexafluorbutan, 2,3-Dibrom-1,1,1,3,4,4,4-heptafluorbutan, 2,3-Dibrom-2-chlor-1,1,1,4,4,4-hexafluorbutan, 1,2-Dibromhexafluorpropan und 1,2-Dichlorhexafluorpropan, wobei Heptafluor-2-brom-propan, 1,2-Dibromhexafluorpropan, 2-Dibrom-1-chlor-trifluorethan besonders bevorzugt sind. Ganz besonders bevorzugt sind 1,2-Dibromhexafluorpropan und Heptafluor-2-brom-propan.

Das molare Verhältnis von Verbindungen der Formel (III) zu Verbindungen der Formel (II) kann pro Äquivalent für n beispielsweise 0,7 bis 1,8 betragen, vorzugsweise 0,9 bis 1,2 und besonders bevorzugt 1,0 bis 1,1.

Die als Ausgangsprodukte verwendeten Verbindungen der Formel (III) sind literaturbekannt oder analog zur Literatur synthetisierbar.

Das erfindungsgemäße Verfahren wird in einem mehrphasigen Reaktionsmediun durchgeführt, das eine wässrige und zumindest eine organischen Phase aufweist.

Besonders geeignete organische Lösungsmittel für mehrphasige Reaktionsmedien sind beispielsweise aliphatische oder aromatische, gegebenenfalls halogenierte Kohlenwasserstoffe wie beispielsweise Benzinfraktionen, Benzol, Toluol, Xylol, Chlorbenzol, Dichlorbenzol, Petrolether, Hexan, Cyclohexan, Dichlormethan, Chloroform oder Tetrachlorkohlenstoff, Ether wie beispielsweise Diethylether, Diisopropylether oder tert.-Butylmethylether, Ketone wie beispielsweise Cyclohexanon, Butanon oder Methyl-isobutyl-keton und Ester wie beispielsweise Essigsäuremethylester oder Essigsäureethylester.

Weiterhin wird das erfindungsgemäße Verfahren in Gegenwart von Phasentransferkatalysator durchgeführt.

Als Phasentransferkatalysatoren sind beispielsweise Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (IV) geeignet,

(Kation⁺)(Anion⁻) (IV)

in der
- (Kation⁺): für substituierte quartäre Ammonium oder Phosphonium-Kationen und
- (Anion⁻): für das Anion einer organischen oder anorganischen Säure steht.

Bevorzugte Phasentransferkatalysatoren sind solche der Formel (IV), in denen (Kation⁺) für Kationen der Formel (V) steht

[Pnyc(C₁-C₁₂-Alkyl)_{q}(C₆-C₁₅-Arylalkyl)ᵣ(C₅-C₁₄-Aryl)_{S}({(C₂-C₆-Alkyl)-O]ᵥ-(C₁-C₆-Alkyl)}ₜ]⁺ (V)

in der
- Pnyc: für Stickstoff oder Phosphor steht und
in denen jeweils (q+r+s+t) = 4 ist.

Bevorzugt steht in Formel (IV) (Anion⁻) für Fluorid, Chlorid, Bromid, Iodid, Acetat, Nitrat, Sulfat, Hydrogensulfat, Tetrafluoroborat, Hexafluorophosphat, Tosylat, und Triflat, besonders bevorzugt für Chlorid, Bromid, Iodid, Sulfat und Hydrogensulfat.

Besonders bevorzugte Phasentransferkatalysatoren sind Tetra-n-butylammoniumiodid, Tetra-n-butylammoniumbromid, Tetra-n-butylammoniumhydrogensulfat, Tetra-n-butylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethylammoniummethylsulfat, Dimethyl-C₁₂/C₁₄-alkyl-benzylammoniumchlorid, Dimethyl-C₁₂/C₁₄-alkyl-benzylammoniumbromid, Triethylbenzylammoniumchlorid, Methyltrioctylammomumchlorid, Trimethylbenzylammoniumchlorid, Tetrakisdiethylaminophosphoniumchlorid, -bromid oder -iodid sowie Tris-[2-(2-methoxyethoxy)-ethyl]-amin, wobei Tetra-n-butylammoniumhydrogensulfat ganz besonders bevorzugt ist.

Die Reaktionstemperatur kann beispielsweise -10°C bis zum Siedepunkt des Reaktionsmediums unter Reaktionsdruck betragen, maximal jedoch 200°C, bevorzugt ist eine Reaktionstemperatur von 0 bis 70°C.

Der Reaktionsdruck kann beispielsweise 0,5 bis 100 bar betragen, Umgebungsdruck ist bevorzugt.

Das erfindungsgemäße Verfahren wird weiterhin in Gegenwart eines Reduktionsmittels und/oder in Gegenwart von Licht mit einer Wellenlänge von 400 nm oder weniger durchgeführt.

Als Reduktionsmittel sind beispielsweise geeignet: Schwefelverbindungen in den gemittelten formalen Oxidationsstufen +III, +IV und +V gegebenenfalls in Mischung mit einem Metall, das ein Standardreduktionspotential von 0 V oder weniger besitzt.

Solche Schwefelverbindungen sind beispielsweise Alkalimetalldithionite, wie Natrium-und Kaliumdithionit oder Schwefeldioxid.

Geeignete Metalle sind beispielweise Mangan, Zink oder Aluminium.

Besonders geeignete Lichtquellen, die Licht mit einer Wellenlänge von 400 nm oder weniger erzeugen, sind alle üblichen UV-Lampen, wie insbesondere Quecksilberdampf-Lampen.

Besonders bevorzugt wird das erfindungsgemäße Verfahren in Gegenwart von Alkalimetalldithionit, ganz besonders bevorzugt in Gegenwart von Natriumdithionit durchgeführt.

Gegebenenfalls aber bevorzugt wird das erfindungsgemäße Verfahren in Gegenwart von Base durchgeführt.

Als Basen sind beispielsweise geeignet: Erdalkali- oder Alkalimetall-hydroxide, - acetate, -phosphate, -hydrogenphosphate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, Ammoniumsalze wie beispielsweise Ammoniumacetat, Ammoniumcarbonat, Amine wie beispielsweise Trimethylamin, Triethylamin, Tributylamin, Di-isopropyl-ethylamin, Tetramethylguanidin, N,N-Dimethylanilin, Diazabicyclooctan (DABCO), Diazabicyclononen (DBN), Diazabicycloundecen (DBU), N-Methylpiperidin und Piperidin, oder aromatische Stickstoffverbindungen wie beispielsweise Pyridin, 2-, 3- und 4-N,N-Dimethylaminopyridin, wobei Alkalimetall-hydroxide, -carbonate und -hydrogencarbonate bevorzugt sind.

Gegebenenfalls können in einem weiteren Reaktionsschritt Verbindungen der Formel (I), in denen der Rest R²R³R⁴C-CR⁵R⁶ noch mindestens ein Chlor- oder Bromatom trägt durch Umsetzung mit ionischem Fluorid zu Verbindungen der Formel (I) umgesetzt werden, in denen besagte Chlor- oder Bromatome durch Fluoratome ersetzt sind.

Ionische Fluoride sind beispielsweise quartäre Ammonium- oder Phosphoniumfluoride sowie Alkalimetallfluoride oder Mischungen der genannten Verbindungen.

Beispiele für Ammonium- oder Phosphoniumfluoride sind solche der Formel (VI),

(Kation⁺)(F⁻) (VI)

in der das (Kation⁺) die unter der Formel (IV) genannte Bedeutung einschließlich deren Vorzugsbereiche besitzt.

Gegebenenfalls können auch Mischungen von Phasentransferkatalysatoren gemäß obiger Definition und/oder Halex-Katalysatoren mit Alkalimetallfluoriden eingesetzt werden.

Bevorzugte Alkalimetallfluoride sind Natrium-, Kalium- und Cäsiumfluorid oder Mischungen davon, besonders bevorzugt ist Kaliumfluorid.

Halex-Katalysatoren sind beispielsweise Tetrakis(dialkylamino)phosphoniumverbindungen (WO 98/05610) oder Verbindungen der Formel (VII), 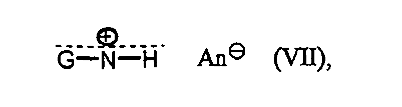 in der
- G: für einen Rest der Formeln (VIIIa) oder (VIIIb) steht 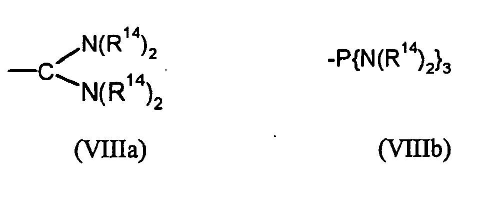 und
- H: unabhängig von G für einen Rest der Formeln (VIIIa), (VIIIb), (VIIIc) oder (VIIId)

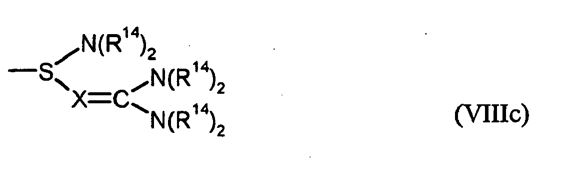

-S[N(R¹⁴)₂]₂ (VIIId)

steht,
wobei
die Reste R¹⁴ jeweils unabhängig voneinander für C₁-C₁₂-Alkyl, C₂-C₁₀-Alkenyl oder C₆-C₁₂-Aryl stehen oder
wobei N(R¹⁴)₂ als Ganzes für einen 3- bis 5-gliedrigen, gesättigten oder ungesättigten Ring stehen kann, oder
wobei die Reste der Formel (VIIIa) und/oder die Gruppe 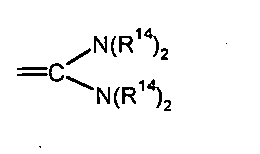 als Ganzes für einen gesättigten oder ungesättigten 4- bis 8-gliedrigen Ring stehen kann, und
- X: für Stickstoff oder Phosphor steht und
- An^{⊖}: für ein Äquivalent eines Anions wie beispielsweise und bevorzugt Chlorid, Bromid, (CH₃)₃SiF₂^{⊖}, HF₂^{⊖}, H₂F₂^{⊖}, Tetrafluoroborat, Hexafluorophosphat, Carbonat oder Sulfat steht.

Die Verbindungen der Formel (VII) sind beispielsweise erhältlich durch Umsetzung von Verbindungen der Formel (IX)

[G-An'] ^{⊕}An^{⊖} (IX),

in der
- G und An^{⊖}: die bei Formel (X) angegebene Bedeutung besitzen und
- An': für Chlor oder Brom und steht,
mit Verbindungen der Formel (X)

HN=G' (X),

in der
- G': hinsichtlich der Anordnung der Atome die bei Formel (X) für G angegebene Bedeutung hat, jedoch 2-bindig ist, wobei die Umsetzung in Gegenwart einer Base erfolgt.

Die Halex-Katalysatoren der Formel (VII) sind beschrieben in DE 101 29 057.

Das molare Verhältnis von ionischem Fluorid zu auszutauschenden Brom- oder Chloratomen in Verbindungen der Formel (I) kann beispielsweise 0,7 bis 5 betragen, vorzugsweise 0,9 bis 2 und besonders bevorzugt 1,1 bis 1,7. Die Menge an ionischem Fluorid ist nach oben prinzipiell nicht begrenzt, größere Mengen sind aber unwirtschaftlich.

Es wurde gefunden, dass üblicherweise Bromatome schneller ausgetauscht werden als Chloratome und die Substitutionsgeschwindigkeit in der Reihe tertiäres, sekundäres, primäres Kohlenstoffatom zunimmt.

Vorzugsweise wird der Halogenaustausch in Gegenwart von organischem Lösungsmittel durchgeführt. Als organische Lösungsmittel sind beispielsweise geeignet: Ketone, wie Aceton, 2-Butanon oder Methyl-isobutyl-keton; Nitrile wie beispielsweise Acetonitril, Propanitril, Benzonitril, Benzylnitril oder Butyronitril; Amide wie beispielsweise N,N-Dimethylformamid, N,N-Dimethylacetamid, N-Methyl-formanilid, N-Methyl-pyrrolidon, N-Methylcaprolactam oder Hexamethylphosphorsäuretriamid; Sulfoxide wie beispielsweise Dimethylsulfoxid, Sulfone wie beispielsweise Tetramethylensulfon, Polyether wie beispielsweise 1,4-Dioxan, Ethylenglykoldimethylether, Ethylenglykoldiethylether, Diethylenglykoldimethylether oder Diethylenglykoldiethylether oder Gemische solcher organischen Lösungsmittel.

Vorzugsweise beträgt der Wassergehalt des Lösungsmittels maximal 1 Gew-%, bevorzugt maximal 0,2 Gew-% und besonders bevorzugt 0,05 Gew.-%. Vorzugsweise wird ein solcher Wassergehalt durch Andestillieren oder Trocknung in an sich bekannter Weise erreicht. Bei Einsatz von Alkalimetallfluoriden wird besonders bevorzugt das Lösungsmittel gleichzeitig in Gegenwart des eingesetzten Alkalimetallfluorids getrocknet bzw. andestilliert.

Die Reaktionstemperatur beim Halogenaustausch kann beispielsweise 60°C bis zum Siedepunkt des eingesetzten Lösungsmittel bei Reaktionsdruck betragen, maximal jedoch 300°C, bevorzugt 110°C bis zum Siedepunkt des eingesetzten Lösungsmittel bei Reaktionsdruck maximal jedoch 200°C.

Der Reaktionsdruck kann beispielsweise 0,5 bis 100 bar betragen, bevorzugt sind 3 bis 25 bar.

Die Reaktionsdauer kann beispielsweise 10 min bis 72 Stunden, bevorzugt 2 bis 12 Stunden betragen.

Die erfindungsgemäß erhältlichen Verbindungen der Formel (I) eignen sich insbesondere in einem Verfahren zur Herstellung von Wirkstoffen wie beispielsweise von Wirkstoffen für Agrochemikalien, wie insbesondere Insektiziden des Aroylharnstofftyps. Besonders bevorzugte Insektizide des Aroylharnstofftyps sind solche, die in EP-A 919 542 und EP-A 936 212 genannt sind.

Überraschend wurde gefunden, dass die durch das erfindungsgemäße Verfahren in besonders vorteilhafter Weise herstellbaren Verbindungen der Formel (XI) als Ausgangsmaterialien für die Herstellung hochwirksamer Insektizide besonders geeignet sind.

In Formel (XI) 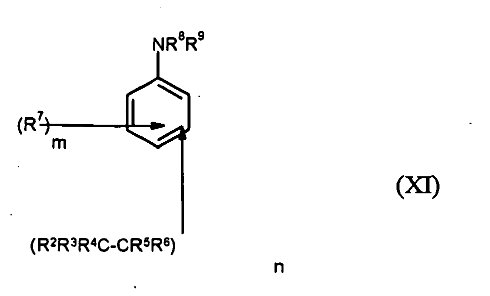 besitzen R², R³, R⁴, R⁵, R⁶, R⁷, R⁸ und R⁹ sowie m und n die gleiche Bedeutungen und Vorzugsbereiche, die unter der Formel (I) bereits genannt wurden, wobei die Auflage gilt, dass entweder
- der Rest R²R³R⁴C-CR⁵R⁶ bezogen auf das Kohlenstoffgerüst ein sekundärer oder tertiärer Rest ist oder
- ein primärer Rest ist, der ausgewählt ist aus der Gruppe 2-Brom-1,1,2,2-tetrafluorethyl, 2-Chlor-1,1,2,2-tetrafluorethyl, 2-Brom-2-chlor-trifluorethyl und 2-Brom-1-chlor-trifluorethyl
und wobei weiterhin Verbindungen ausgenommen sind, in denen R²R³R⁴C-CR⁵R⁶ als Ganzes einen Perfluoralkylrest darstellt.

Weiterhin auch Verbindungen der Formel (XII):

(XIa) **•** (HY)ᵥ

in der
- (XIa): für Verbindungen der Formel (XI) steht, die zumindest eine primäre, sekundäre oder tertiäre Aminofunktion aufweisen und
- v: für eine Zahl zwischen 1 und der Zahl der primären, sekundären oder tertiären Aminofunktionen im Molekül (XIa) steht und
- Y: für ein Anion steht.

Vorzugsweise steht Y für Chlor, Brom und Hydrogensulfat.

Weiterhin sind solche Verbindungen der Formel (XIa) bevorzugt, in denen NR⁸R⁹ als Ganzes für einen primären, sekundären oder tertiären Aminorest steht und die Verbindung der Formel (XIa) keine weiteren primären, sekundären oder tertiären Aminoreste besitzt.

Ein wesentlicher Vorteil des erfindungsgemäßen Verfahrens ist, dass die Verbindungen der Formel (I) in einfacher Weise in hohen Ausbeuten aus gut verfügbaren Edukten hergestellt werden können. *Die* Verbindungen der Formel (XI) und (XII) *stellen* wertvolle Ausgangsprodukte für die Herstellung von Wirkstoffen insbesondere für Agrochemikalien dar.

### Beispiele

### Beispiel 1

### Herstellung von 1,2-Dibrom-hexafluorpropan

Es wurden bei Raumtemperatur 2357 g Brom (760 ml, 14,75 mol) vorgelegt und unter stetigem Rühren Hexafluorpropen bis zur Entfärbung eingeleitet (19 Stunden, 2400 g, 16,00 mol). Das Reaktionsgemisch wurde mit Stickstoff ausgeblasen. Auf diese Weise wurden 4710 g 1,2-Dibrom-hexafluorpropan (95 % d.Th.) erhalten.

### Beispiel 2

### Herstellung von 2-Brom-heptafluorpropan

In einem Autoklaven wurden Tetramethylensulfon (2450 ml) und 352 g Kaliumfluorid (6,05 mol) vorgelegt und der Ansatz durch Abdestillieren von 250 ml Lösungsmittel getrocknet. Anschließend wurden 1250 g 1,2-Dibrom-hexafluorpropan aus Beispiel 1 zugegeben, der Ansatz unter 3 bar Stickstoff gesetzt und auf 125°C aufgeheizt, wobei sich ein Druck von 13,5 bar einstellte. Es wurde bei gleicher Temperatur noch zwei Stunden erhitzt und dann die Temperatur in drei Stunden auf 175°C gesteigert. Es wurde auf 0°C abgekühlt, entspannt und das Produkt aus dem Reaktionsgemisch in eine Kühlfalle destilliert. Auf diese Weise wurden 870 g 2-Brom-heptafluorpropan mit einer Reinheit von 95,8 % erhalten (83 % d.Th.).

### Beispiel 3

### Herstellung von 2-Methyl-4-(1,1,1,2,3,3,3-heptafluor-2-propyl)-anilin

Zu einer Mischung aus 1200 ml Wasser, 250 ml tert.-Butylmethylether, 156,79 g (1,87 mol) Natriumhydrogencarbonat und 22,18 g Tetra-n-butylammoniumhydrogensulfat wurden bei Raumtemperatur zunächst 324,96 g (1,87 mol) Natriumdithionit und anschließend 100 g (0,93 mol) o-Toluidin (2-Methylanilin) gegeben.

Anschließend wurde tropfenweise mit einer Lösung von 489,06 g 2-Brom-1,1,1,2,3,3,3-heptafluorpropan in 200 ml tert.-Butylmethylether versetzt und nach beendeter Zugabe über Nacht bei 30°C nachgerührt. Es wurde falls notwendig mit Natriumcarbonat auf einen pH-Wert von 5 eingestellt und die organische Phase abgetrennt, getrocknet und eingeengt.

Auf diese Weise wurden 250 g (90 % d.Th.) des Produktes mit einer Reinheit von 93 % erhalten.

### Beispiel 4

### Herstellung von N,2-Dimethyl-4-(1,1,1,2,3,3,3-heptafluor-2-propyl)-anilin

Analog zu Beispiel 3 wurde ausgehend von N,2-Dimethylanilin das Produkt in hoher Ausbeute und Reinheit erhalten.

## Patentansprüche

1. Verfahren zur Herstellung von Verbindungen der Formel (I), 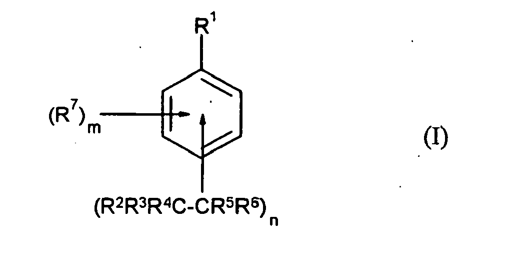 in der
R¹ für C₁-C₁₂-Alkyl, NR⁸R⁹ oder OR¹⁰ steht, wobei R⁸, R⁹ und R¹⁰ jeweils unabhängig voneinander für Wasserstoff, C₁-C₁₂-Alkyl, CO(C₁-C₁₂-Alkyl), CO(C₅-C₁₄-Aryl), CO(C₆-C₁₅-Arylakyl), COO(C₁-C₁₂-Alkyl), COO(C₅-C₁₄-Aryl), COO(C₆-C₁₅-Arylalkyl), COO(C₂-C₁₂-Alkenyl), CONH(C₁-C₁₂-Alkyl), CONH(C₅-C₁₄-Aryl), CONH(C₆-C₁₅-Arylalkyl), CON(C₁-C₁₂-Alkyl)₂, CON(C₅-C₁₄-Aryl)₂, CON(C₆-C₁₅-Arylalkyl)₂ oder C₆-C₁₅-Arylalkyl stehen, oder NR⁸R⁹ als Ganzes für einen cyclischen Rest mit insgesamt 4 bis 16 Kohlenstoffatomen steht und
R², R³, R⁴, R⁵ und R⁶ jeweils unabhängig für Wasserstoff, Fluor, Chlor, Brom oder C₁-C₁₂-Polyfluoralkyl stehen und/oder jeweils zwei der Reste R², R³, R⁴, R⁵ und R⁶ einen oder mehrere cyclische Polyfluoralkylreste mit jeweils insgesamt 4 bis 20 Kohlenstoffatomen bilden, wobei für alle Fälle die Auflage gilt, dass die Summe der Fluoratome am Kohlenstoffatom, das die Bindung zum aromatischen Ring herstellt und den Fluoratomen an dem oder den dazu benachbarten Kohlenstoffatomen mindestens zwei beträgt und
n für eins oder zwei steht und
R⁷ für C₁-C₁₂-Alkyl, C₅-C₁₄-Aryl, C₆-C₁₅-Arylalkyl, Hydroxy, Chlor, Brom, Fluor, Nitro, Cyano, freies oder geschütztes Formyl, C₁-C₁₂-Halogenalkyl, C₅-C₁₄-Aryl, oder Reste der Formeln (IIa) bis (IIf) steht,
A-B-D-E (IIa)
A-E (IIb)
A-SO₂-E (IIc)
A-B-SO₂R¹¹ (IId)
A-SO₃W (IIe)
A-COW (IIf)
in denen unabhängig voneinander
A fehlt oder für einen C₁-C₈-Alkylenrest steht und
B fehlt oder für Sauerstoff, Schwefel oder NR¹² steht,
wobei R¹² Wasserstoff, C₁-C₈-Alkyl, C₆-C₁₅-Arylalkyl oder C₅-C₁₄-Aryl bedeutet und
D für eine Carbonyl-Gruppe steht und
E für R¹³ OR¹³, NHR¹¹ oder N(R¹¹)₂ steht,
wobei R¹³ für C₁-C₈-Alkyl, C₆-C₁₅-Arylalkyl oder C₅-C₁₄-Aryl und
R¹¹ jeweils unabhängig für C₁-C₈-Alkyl, C₆-C₁₅-Arylalkyl oder C₆-C₁₄-Aryl steht oder N(R¹¹)₂ zusammen für einen cyclischen Aminorest mit 4 bis 12 Kohlenstoffatomen steht und
W für OH, NH₂, oder OM steht, wobei M ein Alkalimetallion, ein halbes Äquivalent eines Erdalkalimetallions, ein Ammoniumion oder ein organisches Ammoniumion bedeuten kann oder
jeweils zwei Reste R⁷ zusammen einen cyclischen Rest mit insgesamt 5 bis 12 Kohlenstoffatomen bilden können und
m für eine ganze Zahl von 0 bis 5-n steht,
wobei Aryl jeweils unabhängig einen carbocyclischen aromatischen Rest mit 6 bis 14 Gerüstkohlenstoffatomen oder einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff; Schwefel oder Sauerstoff, substituiert sein können, bedeutet und der carbocyclische aromatische Rest oder heteroaromatische Rest mit bis zu fünf gleichen oder verschiedenen Substituenten pro Cyclus substituiert sein können, die ausgewählt sind aus der Gruppe Chlor, Fluor, C₁-C₁₂-Alkyl, C₁-C₁₂-Perfluoralkyl, COO(C₁-C₈-Alkyl), CON(C₁-C₈-Alkyl)₂, COO(C₁-C₈-Arylalkyl), COO(C₄-C₁₄-Aryl), CO(C₁-C₈-Alkyl), C₅-C₁₅-Arylalkyl oder Tri(C₁-C₆-alkyl)siloxyl,
wobei Alkyl in Alkyl, Alkoxy, Polyfluoralkyl, Perfluoralkyl oder in Arylalkyl jeweils unabhängig einen geradkettigen, cyclischen, verzweigten oder unverzweigten Rest bedeutet, und
wobei Alkylen oder Alkenyl jeweils unabhängig einen geradkettigen, cyclischen, verzweigten oder unverzweigten Alkylen- oder Alkenyl-Rest bedeuten,
**dadurch gekennzeichnet, dass** Verbindungen der Formel (II) 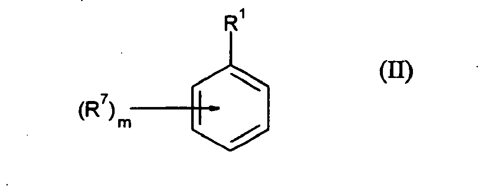 in der
R¹, R⁷ und m die vorstehend genannte Bedeutung besitzen,
mit Verbindungen der Formel (III) umgesetzt werden, 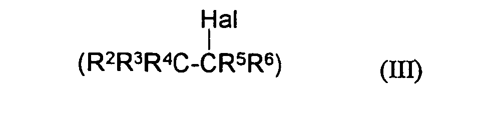 in der
R², R³, R⁴, R⁵ und R⁶ die vorstehend genannte Bedeutung besitzen und
Hal für Brom oder Chlor steht und wobei die Umsetzung
• in einem mehrphasigen Reaktionsmedium, das eine wässrige Phase und zumindest eine, bevorzugt genau eine, organische Phase aufweist und
• in Gegenwart von Phasentransferkatalysator und
• in Gegenwart eines Reduktionsmittels und/oder Licht mit einer Wellenlänge von 400 nm oder weniger erfolgt.

2. Verfahren nach Anspruch 1, **dadurch gekennzeichnet, dass** es in Gegenwart von Base durchgeführt wird.

3. Verfahren nach Anspruch 2, **dadurch gekennzeichnet, dass** als Base Erdalkali- oder Alkalimetall-hydroxide, -acetate, -phosphate, -hydrogenphosphate, - carbonate oder -hydrogencarbonate, Ammoniumsalze, Amine oder aromatische Stickstoffverbindungen eingesetzt werden.

4. Verfahren nach mindestens einem der Ansprüche 1 bis 3, **dadurch gekennzeichnet, dass** R¹ für NR⁷R⁸ steht.

5. Verfahren nach mindestens einem der Ansprüche 1 bis 4, **dadurch gekennzeichnet, dass** R², R³, R⁴ und R⁵ für Wasserstoff, Chlor, Fluor oder C₁-C₄-Perfluoralkyl stehen oder R²R³R⁴C-CR⁵R⁶ als Ganzes für einen cyclischen Polyfluoralkylrest mit insgesamt 4 bis 12 Kohlenstoffatomen steht.

6. Verfahren nach mindestens einem der Ansprüche 1 bis 5, **dadurch gekennzeichnet, dass** n für 1 steht.

7. Verfahren nach mindestens einem der Ansprüche 1 bis 6, **dadurch gekennzeichnet, dass** R⁷ jeweils unabhängig für C₁-C₄-Alkyl, Chlor, Fluor, Nitro, Cyano oder C₁-C₄-Alkoxy steht.

8. Verfahren nach mindestens einem der Ansprüche 1 bis 7, **dadurch gekennzeichnet, dass** m für 1 oder 2 steht.

9. Verfahren nach mindestens einem der Ansprüche 1 bis 8, **dadurch gekennzeichnet, dass** 2-Methyl-4-(heptafluor-2-propyl)-anilin, N,2-Dimethyl-4-(1,1,1,2,3,3,3-heptafluor-2-propyl)-anilin, 2-Methyl-4-(1-brom-1,1,2,3,3,3-hexafluor-2-propyl)-anilin, 2-Methyl-4-(2-brom-1,1,2,2-tetrafluorethyl)-anilin, sind 2-Methyl-4-(2-chlor-1,1,2,2-tetrafluorethyl)-anilin, sind 2-Methyl-4-(1-chlor-1,1,2,3,3,3-hexafluor-2-propyl)-anilin, sind 2-Methyl-4-(2-brom-2-chlor-trifluorethyl)-anilin, 2-Methyl-4-(2-brom-1-chlor-trifluorethyl)-anilin, 2-Methyl-4-(3-brom-2,3-dichlor-1,1,1,4,4,4-hexafluor-2-butyl)-anilin, 2-Methyl-4-(2-chlor-3,3,4,4-tetrafluor-cyclobutyl)-anilin, 2-Methyl-4-(2-chlor-3,3,4,4,5,5-hexafluor-cyclopentyl)-anilin, 2-Methyl-4-(2-brom-3,3,4,4-tetrafluor-cyclobutyl)-anilin, 2-Methyl-4-(2-brom-3,3,4,4,5,5-hexafluor-cyclopentyl)-anilin, Essigsäure-2-methyl-4-(1-brom-1,1,2,3,3,3-hexafluor-2-propyl)-anilid, 2-Methyl-4-(2-brom-1,1,1,2,3,4,4,4-octafluor-3-butyl)-anilin oder 2-Methyl-4-(2-brom-2,3,3,4,4,5,5-octafluorcyclo-1-pentyl)-anilin hergestellt werden.

10. Verfahren nach mindestens einem der Ansprüche 1 bis 9, **dadurch gekennzeichnet, dass** als Verbindungen der Formel (III) Heptafluor-2-brom-propan, Heptafluor-2-chlor-propan, 1,2-Dibromtetrafluorethan, 1,2-Dibrom-1-chlortrifluorethan, 2,3-Dibrom-octafluorbutan, 2,3-Dibrom-2,3-dichlorhexafluorbutan, 2,3-Dibrom-2,3-dichlorhexafluorbutan, 2,3-Dibrom-1,1,1,3,4,4,4-heptafluorbutan, 2,3-Dibrom-2-chlor-1,1,1,4,4,4-hexafluorbutan, 1,2-Dibromhexafluorpropan und 1,2-Dichlorhexafluorpropan eingesetzt werden.

11. Verfahren nach mindestens einem der Ansprüche 1 bis 10, **dadurch gekennzeichnet, dass** als organische Lösungsmittel für mehrphasige Reaktionsmedien aliphatische oder aromatische, gegebenenfalls halogenierte Kohlenwasserstoffe, Ether, Ketone und Ester eingesetzt werden.

12. Verfahren nach mindestens einem der Ansprüche 1 bis 11, **dadurch gekennzeichnet, dass** als Phasentransferkatalysatoren Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (IV) eingesetzt werden,
(Kation⁺)(Anion⁻) (IV)
in der
(Kation⁺) für substituierte quartäre Ammonium oder Phosphonium-Kationen und
(Anion⁻) für das Anion einer organischen oder anorganischen Säure steht.

13. Verfahren nach Anspruch 12, **dadurch gekennzeichnet, dass** als Phasentransferkatalysatoren solche der Formel (IV) eingesetzt werden, in denen (Kation⁺) für Kationen der Formel (V) steht
[Pnyc(C₁-C₁₈-Alkyl)_{q}(C₆-C₁₅-Arylalkyl)ᵣ(C₅-C₁₄-Aryl)_{S}({(C₂-C₆-Alkyl)-O]ᵥ-(C₁-C₆-Alkyl)}ₜ)]⁺ (V)
in der
Pnyc für Stickstoff oder Phosphor steht und
in denen jeweils (q+r+s+t) = 4 ist.

14. Verfahren nach mindestens einem der Ansprüche 1 bis 13, **dadurch gekennzeichnet, dass** die Reaktionstemperatur -10°C bis zum Siedepunkt des Reaktionsmediums unter Reaktionsdruck, maximal jedoch 200°C beträgt und der Reaktionsdruck 0,5 bis 100 bar beträgt.

15. Verfahren nach mindestens einem der Ansprüche 1 bis 14, **dadurch gekennzeichnet, dass** als Reduktionsmittel Schwefelverbindungen in den gemittelten formalen Oxidationsstufen +III, +IV und +V gegebenenfalls in Mischung mit einem Metall eingesetzt werden, das ein Standardreduktionspotential von 0 V oder weniger besitzt.

16. Verfahren nach mindestens einem der Ansprüche 1 bis 15, **dadurch gekennzeichnet, dass** Alkalimetalldithionite eingesetzt werden.

17. Verfahren nach mindestens einem der Ansprüche 1 bis 16, **dadurch gekennzeichnet, dass** in einem Folgeschritt Verbindungen der Formel (I), in denen der Rest R²R³R⁴C-CR⁵R⁶ noch mindestens ein Chlor- oder Brom-atom trägt durch Umsetzung mit ionischem Fluorid zu Verbindungen der Formel (I) umgesetzt werden, in denen mindestens ein Chlor- oder Brom-atom durch ein Fluoratom ersetzt sind.

18. Verfahren nach Anspruch 17, **dadurch gekennzeichnet, dass** als ionisches Fluorid quartäre Ammonium- oder Phosphoniumfluoride sowie Alkalimetallfluoride oder Mischungen der genannten Verbindungen oder Mischungen von Phasentransferkatalysatoren und/oder Halex-Katalysatoren mit Alkalimetallfluoriden eingesetzt werden.

19. Verfahren nach mindestens einem der Ansprüche 17 und 18, **dadurch gekennzeichnet, dass** der Halogenaustausch in Gegenwart von organischem Lösungsmittel durchgeführt wird, wobei die Lösungsmittel Ketone, Nitrile, Amide, Sulfoxide, Sulfone, Polyether oder Gemischen solcher organischen Lösungsmittel sind.

## Claims

1. Process for preparing compounds of the formula (I) 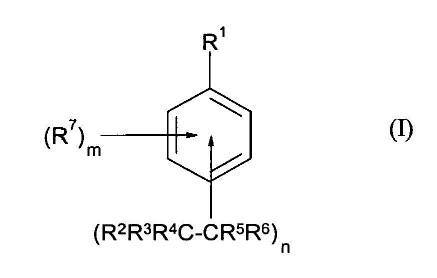 in which
R¹ is C₁-C₁₂-alkyl, NR⁸R⁹ or OR¹⁰, where R⁸, R⁹ and R¹⁰ are each independently hydrogen, C₁-C₁₂-alkyl, CO(C₁-C₁₂-alkyl), CO(C₅-C₁₄-aryl), CO(C₆-C₁₅-arylalkyl), COO(C₁-C₁₂-alkyl), COO(C₅-C₁₄-aryl), COO(C₆-C₁₅-arylalkyl), COO(C₂-C₁₂-alkenyl), CONH(C₁-C₁₂-alkyl), CONH(C₅-C₁₄-aryl), CONH(C₆-C₁₅-arylalkyl), CON(C₁-C₁₂-alkyl)₂, CON(C₅C₁₄-aryl)₂, CON(C₆-C₁₅-arylalkyl)₂ or C₆-C₁₅-arylalkyl, or NR⁸R⁹ as a whole is a cyclic radical having a total of 4 to 16 carbon atoms and
R², R³, R⁴, R⁵ and R⁶ are each independently hydrogen, fluorine, chlorine, bromine or C₁-C₁₂-polyfluoroalkyl, and/or at least two of the R², R³, R⁴, R⁵ and R⁶ radicals each form one or more cyclic polyfluoroalkyl radicals each having a total of 4 to 20 carbon atoms, with the proviso in all cases that the sum of the fluorine atoms on the carbon atom which forms the bond to the aromatic ring and the fluorine atoms at the adjacent carbon atom or atoms is at least two and
n is one or two, and
R⁷ is C₁-C₁₂-alkyl, C₅-C₁₄-aryl, C₆-C₁₅-arylalkyl, hydroxyl, chlorine, bromine, fluorine, nitro, cyano, free or protected formyl, C₁-C₁₂-haloalkyl, or radicals of the formulae (IIa) to (IIf),
A-B-D-E (IIa)
A-E (IIb)
A-SO₂-E (IIc)
A-B-SO₂R¹¹ (IId)
A-SO₃W (IIe)
A-COW (IIf)
in which, each independently,
A is absent or is a C₁-C₈-alkylene radical and
B is absent or is oxygen, sulphur or NR¹²,
where R¹² is hydrogen, C₁-C₈-alkyl, C₆-C₁₅-arylalkyl or C₅-C₁₄-aryl, and
D is a carbonyl group and
E is R¹³, OR¹³, NHR¹¹ or N(R¹¹)₂
where R¹³ is C₁-C₈-alkyl, C₆-C₁₅-arylalkyl or C₅-C₁₄-aryl, and
R¹¹ is in each case independently C₁-C₈-alkyl, C₆-C₁₅-arylalkyl or C₆-C₁₄-aryl, or N(R¹¹)₂ together is a cyclic amino radical having 4 to 12 carbon atoms and
W is OH, NH₂ or OM where M may be an alkali metal ion, half an equivalent of an alkaline earth metal ion, an ammonium ion or an organic ammonium ion, or
two R⁷ radicals together, in each case, may form a cyclic radical having a total of 5 to 12 carbon atoms, and
m is an integer from 0 to 5-n,
where aryl is in each case independently a carbocyclic aromatic radical having 6 to 14 skeleton carbon atoms or a heteroaromatic radical having 5 to 14 skeleton carbon atoms, in which no, one, two or three skeleton carbon atoms per cycle, but at least one skeleton carbon atom in the entire molecule, may be substituted by heteroatoms selected from the group of nitrogen, sulphur and oxygen, and the carbocyclic aromatic radical or heteroaromatic radical may be substituted by up to five identical or different substituents per cycle which are selected from the group of chlorine, fluorine, C₁-C₁₂-alkyl, C₁-C₁₂-perfluoroalkyl, COO(C₁-C₈-alkyl), CON(C₁-C₈-alkyl)₂, COO(C₁-C₈-arylalkyl), COO(C₄-C₁₄-aryl), CO(C₁-C₈-alkyl), C₅-C₁₅-arylalkyl or tri(C₁-C₆-alkyl)siloxyl,
where alkyl in alkyl, alkoxy, polyfluoroalkyl, perfluoroalkyl or in arylalkyl is in each case independently a straight-chain, cyclic, branched or unbranched radical, and
where alkylene or alkenyl is in each case independently a straight-chain, cyclic, branched or unbranched alkylene or alkenyl radical,
**characterized in that** compounds of the formula (II) 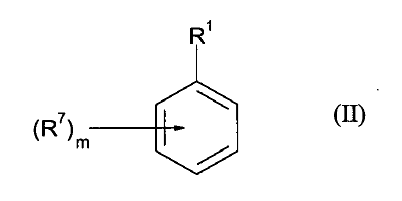 in which
R¹, R⁷ and m are each as defined above
are reacted with compounds of the formula (III) 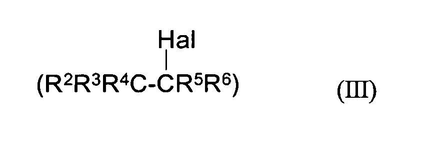 in which
R², R³, R⁴, R⁵ and R⁶ are each as defined above and
Hal is bromine or chlorine, and the reaction is effected
• in a multiphasic reaction medium which has one aqueous phase and at least one, preferably exactly one, organic phase and
• in the presence of phase transfer catalyst and
• in the presence of a reducing agent and/or light having a wavelength of 400 nm or less.

2. Process according to Claim 1, **characterized in that** it is carried out in the presence of base.

3. Process according to Claim 2, **characterized in that** the base used is an alkali metal or alkaline earth metal hydroxide, acetate, phosphate, hydrogenphosphate, carbonate or hydrogencarbonate, ammonium salt, amine or aromatic nitrogen compound.

4. Process according to at least one of Claims 1 to 3, **characterized in that** R¹ is NR⁷R⁸.

5. Process according to at least one of Claims 1 to 4, **characterized in that** R², R³, R⁴ and R⁵ are each hydrogen, chlorine, fluorine or C₁-C₄-perfluoroalkyl, or R²R³R⁴C-CR⁵R⁶ as a whole is a cyclic polyfluoroalkyl radical having a total of 4 to 12 carbon atoms.

6. Process according to at least one of Claims 1 to 5, **characterized in that** n is 1.

7. Process according to at least one of Claims 1 to 6, **characterized in that** R⁷ is in each case independently C₁-C₄-alkyl, chlorine, fluorine, nitro, cyano or C₁-C₄-alkoxy.

8. Process according to at least one of Claims 1 to 7, **characterized in that** m is 1 or 2.

9. Process according to at least one of Claims 1 to 8, **characterized in that** 2-methyl-4-(heptafluoro-2-propyl)aniline, N,2-dimethyl-4-(1,1,1,2,3,3,3-heptafluoro-2-propyl)aniline, 2-methyl-4-(1-bromo-1,1,2,3,3,3-hexafluoro-2-propyl)aniline, 2-methyl-4-(2-bromo-1,1,2,2-tetrafluoroethyl)aniline, 2-methyl-4-(2-chloro-1,1,2,2-tetrafluoroethyl)aniline, 2-methyl-4-(1-chloro-1,1,2,3,3,3-hexafluoro-2-propyl)aniline, 2-methyl-4-(2-bromo-2-chlorotrifluoroethyl)aniline, 2-methyl-4-(2-bromo-1-chlorotrifluoro-ethyl)aniline, 2-methyl-4-(3-bromo-2,3-dichloro-1,1,1,4,4,4-hexafluoro-2-butyl)aniline, 2-methyl-4-(2-chloro-3,3,4,4-tetrafluorocyclobutyl)aniline, 2-methyl-4-(2-chloro-3,3,4,4,5,5-hexafluorocyclopentyl)aniline, 2-methyl-4-(2-bromo-3,3,4,4-tetrafluorocyclobutyl)aniline, 2-methyl-4-(2-bromo-3,3,4,4,5,5-hexafluorocyclopentyl)aniline, 2-methyl-4-(1-bromo-1,1,2,3,3,3-hexafluoro-2-propyl)acetanilide, 2-methyl-4-(2-bromo-1,1,1,2,3,4,4,4-octafluoro-3-butyl)aniline or 2-methyl-4-(2-bromo-2,3,3,4,4,5,5-octafluorocyclo-1-pentyl)aniline are prepared.

10. Process according to at least one of Claims 1 to 9, **characterized in that** the compounds of the formula (III) used are heptafluoro-2-bromopropane, heptafluoro-2-chloropropane, 1,2-dibromotetrafluoroethane, 1,2-dibromo-1-chlorotrifluoroethane, 2,3-dibromooctafluorobutane, 2,3-dibromo-2,3-dichlorohexafluorobutane, 2,3-dibromo-2,3-dichlorohexafluorobutane, 2,3-dibromo-1,1,1,3,4,4,4-heptafluorobutane, 2,3-dibromo-2-chloro-1,1,1,4,4,4-hexafluorobutane, 1,2-dibromohexafluoropropane and 1,2-dichlorohexafluoropropane.

11. Process according to at least one of Claims 1 to 10, **characterized in that** the organic solvent used for multiphasic reaction media is an aliphatic or aromatic, optionally halogenated hydrocarbon, ether, ketone, or ester.

12. Process according to at least one of Claims 1 to 11, **characterized in that** the phase transfer catalysts used are crown ethers such as 18-crown-6, 12-crown-4, dibenzo-18-crown-6 or dibenzo-12-crown-4, cryptands such as cryptand[2.2.2] or podands such as polyglycol ethers or those of the formula (IV),
(cation⁺)(anion⁻) (IV)
in which
(cation⁺) is a substituted quaternary ammonium or phosphonium cation and
(anion⁻) is the anion of an organic or inorganic acid.

13. Process according to Claim 12, **characterized in that** the phase transfer catalysts used are those of the formula (IV) in which (cation⁺) is a cation of the formula (V)
[pnic(C₁-C₁₂-alkyl)_{q}(C₆-C₁₅arylalkyl)ᵣ(C₅-C₁₄-aryl)ₛ({(C₂-C₆-alkyl)-O]ᵥ-(C₁-C₆-alkyl)}ₜ]⁺ (V)
in which
pnic is nitrogen or phosphorus and
in which in each case (q+r+s+t) = 4.

14. Process according to at least one of Claims 1 to 13, **characterized in that** the reaction temperature is -10°C up to the boiling point of the reaction medium under reaction pressure, but a maximum of 200°C, the reaction pressure being 0.5 to 100 bar.

15. Process according to at least one of Claims 1 to 14, **characterized in that** the reducing agent used is a sulphur compound in the average formal oxidation states +III, +IV and +V, optionally in a mixture with a metal which has a standard reduction potential of 0 V or less.

16. Process according to at least one of Claims 1 to 15, **characterized in that** alkali metal dithionites are used.

17. Process according to at least one of Claims 1 to 16, **characterized in that**, in a subsequent step, compounds of the formula (I) in which the R²R³R⁴C-CR⁵R⁶ radical also bears at least one chlorine or bromine atom are reacted with ionic fluoride to give compounds of the formula (I) in which at least one chlorine or bromine atom has been replaced by a fluorine atom.

18. Process according to Claim 17, **characterized in that** the ionic fluorides used are quaternary ammonium fluorides or phosphonium fluorides, or else alkali metal fluorides or mixtures of the compounds mentioned or mixtures of phase transfer catalysts and/or halex catalysts with alkali metal fluorides.

19. Process according to at least one of Claims 17 and 18, **characterized in that** the halogen exchange is carried out in the presence of organic solvent, the solvent being selected from ketones, nitriles, amides, sulphoxides, sulphones, polyethers or mixtures of such organic solvents.

## Revendications

1. Procédé pour la préparation de composés de formule (I), 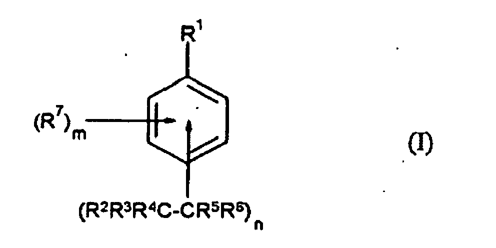 dans laquelle
R¹ représente un groupe alkyle en C₁-C₁₂, NR⁸R⁹ ou OR¹⁰, R⁸, R⁹ et R¹⁰ représentant chacun indépendamment un atome d'hydrogène, un groupe alkyle en C₁-C₁₂, CO-[alkyle (C₁-C₁₂)], CO-[aryle (C₅-C₁₄)], CO-[arylalkyle(C₆-C₁₅)], COO-[alkyle (C₁-C₁₂)], COO- [aryle (C₅-C₁₄)], COO-[arylalkyle (C₆-C₁₅)], COO- [alcényle (C₂-C₁₂)], CONH- [alkyle(C₁-C₁₂)], CONH- [aryle (C₅-C₁₄)], CONH [arylalkyle (C₆-C₁₅)], CON- [alkyle(C₁-C₁₂)]₂, CON- [aryle (C₅-C₁₄)]₂, CON- [arylalkyle (C₆-C₁₅)]₂ ou arylalkyle en C₆-C₁₅, ou NR⁸R⁹ représentant dans son ensemble un radical cyclique ayant au total de 4 à 16 atomes de carbone et
R², R³, R⁴, R⁵ et R⁶ représentent chacun, indépendamment les uns des autres, un atome d'hydrogène, de fluor, de chlore ou de brome ou un groupe polyfluoroalkyle en C₁-C₁₂ et/ou chaque fois deux des radicaux R², R³, R⁴, R⁵ et R⁶ forment un ou plusieurs radicaux polyfluoroalkyle cycliques ayant chacun au total de 4 à 20 atomes de carbone, pour tous les cas la somme des atomes de fluor sur l'atome de carbone produisant la liaison au cycle aromatique et des atomes de fluor sur l'atome de carbone ou les atomes de carbones voisins de celui-ci devant être au moins égale à deux et
n représente un ou deux et
R⁷ représente un groupe alkyle en C₁-C₁₂, aryle en C₅-C₁₄, arylalkyle en C₆-C₁₅, hydroxy, un atome de chlore, de brome ou de fluor, un groupe nitro, cyano, formyle libre ou protégé, halogénoalkyle en C₁-C₁₂, aryle en C₅-C₁₄ ou des radicaux de formules (IIa) à (IIf),
A-B-D-E (IIa)
A-E (IIb)
A-SO₂- (IIc)
A-B-SO₂R¹¹ (IId)
A-SO₃W (IIe)
A-COW (IIf)
dans lesquelles, indépendamment les uns des autres,
A est absent ou représente un radical alkylène en C₁-C₈ et
B est absent ou représente un atome d'oxygène ou de soufre ou NR¹²,
R¹² représentant un atome d'hydrogène, un groupe alkyle en C₁-C₈, arylalkyle en C₆-C₁₅ ou aryle en C₅-C₁₄ et
D représente un groupe carbonyle et
E représente R¹³, OR¹³, NHR¹¹ ou N(R¹¹)₂,
R¹³ représentant un groupe alkyle en C₁-C₈, arylalkyle en C₆-C₁₅ ou aryle en C₅-C₁₄ et
R¹¹ représentant, chacun indépendamment, un groupe alkyle en C₁-C₈, arylalkyle en C₆-C₁₅ ou aryle en C₆-C₁₄, ou N(R¹¹)₂ représentant dans son ensemble un radical amino cyclique ayant de 4 à 12 atomes de carbone et
W représente OH, NH₂ ou OM, M pouvant représenter un ion de métal alcalin, un demi-équivalent d'un ion de métal alcalino-terreux, un ion ammonium ou un ion ammonium organique ou
chaque fois deux radicaux R⁷ peuvent former ensemble un radical cyclique ayant au total de 5 à 12 atomes de carbone et
m représente un nombre entier allant de 0 à 5-n,
aryle signifiant chaque fois indépendamment un radical aromatique carbocyclique ayant de 6 à 14 atomes de carbone dans le squelette ou un radical hétéroaromatique ayant de 5 à 14 atomes de carbone du squelette, dans lesquels aucun, un, deux ou trois atomes de carbone du squelette par cycle, mais au moins un atome de carbone du squelette dans toute la molécule, peuvent être remplacés par des hétéroatomes choisis dans l'ensemble constitué par les atomes d'azote, de soufre et d'oxygène, et le radical aromatique carbocyclique ou le radical hétéroaromatique pouvant être substitués par jusqu'à cinq substituants identiques ou différents par cycle, choisis dans l'ensemble constitué par les atomes de chlore et de fluor et les groupes alkyle en C₁-C₁₂, perfluoroalkyle en C₁-C₁₂, COO-[alkyle (C₁-C₈)], CON-[alkyle(C₁-C₈)]₂, COO- [arylalkyle(C₁-C₈)], COO- [aryle(C₄-C₁₄)], CO-[alkyl (C₁-C₈)], arylalkyle (C₅-C₁₅) et tri[alkyl(C₁-C₆)]siloxy,
le fragment alkyle dans les groupes alkyle, alcoxy, polyfluoroalkyle, pefluoroalkyle ou arylalkyle signifiant chaque fois indépendamment un radical à chaîne droite, cyclique, ramifié ou non ramifié, et
le groupe alkylène ou alcényle signifiant chaque fois indépendamment un radical alkylène ou alcényle à chaîne droite, cyclique, ramifié ou non ramifié,
**caractérisé en ce qu'**on fait réagir des composés de formule (II) 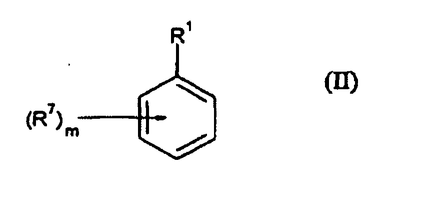 dans laquelle
R¹, R⁷ et m ont les significations données précédemment,
avec des composés de formule (III), 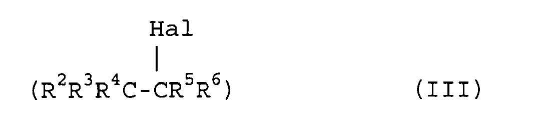 dans laquelle
R², R³, R⁴, R⁵ et R⁶ ont les significations données précédemment et
Hal représente le brome ou le chlore,
et la réaction
• étant effectuée dans un milieu réactionnel en plusieurs phases comportant une phase aqueuse et au moins une, de préférence juste une, phase organique et
• en présence d'un catalyseur de transfert de phase et
• en présence d'un réducteur et/ou de lumière ayant une longueur d'onde de 400 nm ou moins.

2. Procédé selon la revendication 1, **caractérisé en ce qu'**il est effectué en présence d'une base.

3. Procédé selon la revendication 2, **caractérisé en ce qu'**on utilise comme base des hydroxydes, acétates, phosphates, hydrogénophosphates, carbonates ou hydrogénocarbonates de métaux alcalins ou alcalino-terreux, des sels d'ammonium, des amines ou des composés azotés aromatiques.

4. Procédé selon au moins l'une des revendications 1 à 3, **caractérisé en ce que** R¹ représente NR⁷R⁸.

5. Procédé selon au moins l'une des revendications 1 à 4, **caractérisé en ce que** R², R³, R⁴ et R⁵ représentent un atome d'hydrogène, de chlore ou de fluor, ou un groupe perfluoroalkyle en C₁-C₄ ou R²R³R⁴C-CR⁵R⁶ représente dans son ensemble un radical polyfluoroalkyle cyclique ayant au total de 4 à 12 atomes de carbone.

6. Procédé selon au moins l'une des revendications 1 à 5, **caractérisé en ce que** n représente 1.

7. Procédé selon au moins l'une des revendications 1 à 6, **caractérisé en ce que** R⁷ représente chaque fois indépendamment un atome de chlore ou de fluor ou un groupe alkyle en C₁-C₄, nitro, cyano ou alcoxy en C₁-C₄.

8. Procédé selon au moins l'une des revendications 1 à 7, **caractérisé en ce que** m représente 1 ou 2.

9. Procédé selon au moins l'une des revendications 1 à 8, **caractérisé en ce qu'**on prépare la 2-méthyl-4-(heptafluoro-2-propyl)-aniline, la N,2-diméthyl-4-(1,1,1,2,3,3,3-heptafluoro-2-propyl)-aniline, la 2-méthyl-4-(1-bromo-1,1,2,3,3,3-hexafluoro-2-propyl)-aniline, la 2-méthyl-4-(2-bromo-1,1,2,2-tetrafluoréthyl)-aniline, la 2-méthyl-4-(2-chloro-1,1,2,2-tétrafluoroéthyl)-aniline, la 2-méthyl-4-(1-chloro-1,1,2,3,3,3-hexafluoro-2-propyl)-aniline, la 2-méthyl-4-(2-bromo-2-chlorotrifluoroéthyl)-aniline, la 2-méthyl-4-(2-bromo-1-chloro-trifluoroéthyl)-aniline, la 2-méthyl-4-(3-bromo-2,3-dichloro-1,1,1,4,4,4-hexafluoro-2-butyl)-aniline, la 2-méthyl-4-(2-chloro-3,3,4,4-tétrafluoro-cyclobutyl)-aniline, la 2-méthyl-4-(2-chloro-3,3,4,4,5,5-hexafluoro-cyclopentyl)-aniline, la 2-méthyl-4-(2-bromo-3,3,4,4-tétrafluoro-cyclobutyl)-aniline, la 2-méthyl-4-(2-bromo-3,3,4,4,5,5-hexafluoro-cyclopentyl)-aniline, le 2-méthyl-4-(1-bromo-1,1,2,3,3,3-hexafluoro-2-propyl)-acétanilide, la 2-méthyl-4-(2-bromo-1,1,1,2,3,4,4,4-octafluoro-3-butyl)-aniline ou la 2-méthyl-4-(2-bromo-2,3,3,4,4,5,5-octafluorocyclo-1-pentyl)-aniline.

10. Procédé selon au moins l'une des revendications 1 à 9, **caractérisé en ce qu'**on utilise comme composés de formule (III) l'heptafluoro-2-bromo-propane, l'heptafluoro-2-chloro-propane, le 1,2-dibromotétrafluoroéthane, le 1,2-dibromo-1-chloro-trifluoroéthane, le 2,3-dibromo-octafluorobutane, le 2,3-dibromo-2,3-dichlorohexafluorobutane, le 2,3-dibromo-2,3-dichlorohexafluorobutane, le 2,3-dibromo-1,1,1,3,4,4,4-heptafluorobutane, le 2,3-dibromo-2-chloro-1,1,1,4,4,4-hexafluorobutane, le 1,2-dibromohexafluoropropane et le 1,2-dichlorohexafluoropropane.

11. Procédé selon au moins l'une des revendications 1 à 10, **caractérisé en ce qu'**on utilise comme solvant organique pour des milieux réactionnels en deux phases des hydrocarbures aliphatiques ou aromatiques, éventuellement halogénés, des éthers, des cétones et des esters.

12. Procédé selon au moins l'une des revendications 1 à 11, **caractérisé en ce qu'**on utilise en tant que catalyseurs de transfert de phase des éthers-couronnes, tels que l'éther-18-couronne-6, l'éther 12-couronne-4, l'éther dibenzo-18-couronne-6 ou l'éther dibenzo-12-couronne-4, des cryptands tels que le cryptand[2.2.2] ou des podands tels des éthers de polyglycol ou ceux de formule (IV),
(cation⁺) (anion⁻) (IV)
dans laquelle
(cation⁺) représente des cations phosphonium ou ammonium quaternaire substitués et
(anion⁻) représente l'anion d'un acide organique ou inorganique.

13. Procédé selon la revendication 12, **caractérisé en ce qu'**on utilise en tant que catalyseurs de transfert de phase ceux de formule (IV), dans lesquels (cation⁺) représente des cations de formule (V)
[Pnyc(alkyl(C₁-C₁₂))_{q}(arylakyl(C₆-C₁₅))ᵣ(aryl-(C₅-C₁₄))ₛ({(alkyl(C₂-C₆))-O]ᵥ-(alkyl(C₁-C₆))}ₜ)]⁺ (V)
dans laquelle
Pnyc représente un atome d'azote ou de phosphore et
dans lesquels (q+r+s+t) = 4.

14. Procédé selon au moins l'une des revendications 1 à 13, **caractérisé en ce que** la température de réaction est dans la plage allant de -10°C au point d'ébullition du milieu réactionnel sous la pression de la réaction, mais au maximum de 200°C et la pression de la réaction est dans la plage de 0,5 à 100 bars.

15. Procédé selon au moins l'une des revendications 1 à 14, **caractérisé en ce qu'**on utilise en tant que réducteur des composés soufrés aux degrés d'oxydation moyens selon la formule de +III, +IV et +V, éventuellement en mélange avec un métal qui possède un potentiel de réduction standard de 0 V ou moins.

16. Procédé selon au moins l'une des revendications 1 à 15, **caractérisé en ce qu'**on utilise des dithionites de métaux alcalins.

17. Procédé selon au moins l'une des revendications 1 à 16, **caractérisé en ce que** dans une étape suivante on convertit des composés de formule (I), dans lesquels R²R³R⁴C-CR⁵R⁶ porte encore au moins un atome de chlore ou de brome, par mise en réaction avec un fluorure ionique, en composés de formule (I) dans lesquels au moins un atome de chlore ou de brome est remplacé par un atome de fluor.

18. Procédé selon la revendication 17, **caractérisé en ce qu'**on utilise comme fluorure ionique des fluorures d'ammonium ou de phosphonium quaternaire ainsi que des fluorures de métaux alcalins ou des mélanges des composés cités ou des mélanges de catalyseurs de transfert de phase et/ou des catalyseurs Halex avec des fluorures de métaux alcalins.

19. Procédé selon la revendication 17 ou 18, **caractérisé en ce que** l'échange d'halogène est effectué en présence d'un solvant organique, les solvants étant des cétones, des nitriles, des amides, des sulfoxydes, des sulfones, des polyéthers ou des mélanges de tels solvants organiques.
